# EUROPEAN PATENT APPLICATION

(11) **EP 3 809 420 A1**
(43) Date of publication of application: **21.04.2021**
(21) Application number: 20150573.2
(22) Date of filing: 07.01.2020
(51) Int. Cl.: G16H 50/20, G16H 50/70

(54) **SYSTEM AND METHOD FOR PHYSIOLOGICAL PARAMETER ESTIMATIONS**

(30) Priority: 15.10.2019 US 201962915179 P
(71) Applicant: Koninklijke Philips N.V., 5656 AG Eindhoven (NL)
(72) Inventor: HENDRIKS, Cornelis Petrus, 5656 AE Eindhoven (NL); COX, Lieke Gertruda Elisabeth, 5656 AE Eindhoven (NL); VAN BEERS, Paulus René Maria, 5656 AE Eindhoven (NL); LAVEZZO, Valentina, 5656 AE Eindhoven (NL); BULUT, Murtaza, 5656 AE Eindhoven (NL)
(74) Representative: Steenbeek, Leonardus Johannes

(57) **Abstract**

A means is provided for implementing a digital twin to provide estimated measurements of one or more physiological or anatomical parameters but in a way that requires less local storage space and local processing capacity. Embodiments are based on constructing and implementing a lean digital twin model or dataset by storing results of a number of full digital model simulations in advance and then using these pre-stored simulation results to provide physiological parameter estimations, in place of the full digital twin model.

## Description

### FIELD OF THE INVENTION

This invention relates to a system and method for use in deriving estimations of physiological or anatomical parameters for a patient, in particular making use of simulations performed by a digital twin of the patient.

### BACKGROUND OF THE INVENTION

A recent development in technology is the so-called digital twin concept. In this concept, a digital representation (the digital twin) of a physical system is provided and connected to its physical counterpart, for example through the Internet of things as explained in US 2017/286572 A1. Through this connection, the digital twin typically receives data pertaining to the state of the physical system, such as sensor readings or the like, based on which the digital twin can predict the actual or future status of the physical system, e.g. through simulation, as well as analyze or interpret a status history of the physical twin. In case of electromechanical systems, this for example may be used to predict the end-of-life of components of the system, thereby reducing the risk of component failure as timely replacement of the component may be arranged based on its end-of-life as estimated by the digital twin.

Such digital twin technology is also becoming of interest in the medical field, as it provides an approach to more efficient medical care provision. For example, the digital twin may be built using imaging data of the patient, e.g. a patient suffering from a diagnosed medical condition as captured in the imaging data, as for instance is explained by Dr Vanessa Diaz in https://www.wareable.com/health-and-wellbeing/doctor-virtual-twin-digital-patient-ucl-887 as retrieved from the Internet on 29 October 2018. Such a digital twin may serve a number of purposes. Firstly, the digital twin rather than the patient may be subjected to a number of virtual tests, e.g. treatment plans, to determine which treatment plan is most likely to be successful to the patient. This therefore reduces the number of tests that physically need to be performed on the actual patient.

The digital twin of the patient for instance further may be used to predict the onset, treatment or development of such medical conditions of the patient using a patient-derived digital model, e.g. a digital model that has been derived from medical image data of the patient. In this manner, the medical status of a patient may be monitored without the routine involvement of a medical practitioner, e.g. thus avoiding periodic routine physical checks of the patient. Instead, only when the digital twin predicts a medical status of the patient indicative of the patient requiring medical attention based on the received sensor readings may the digital twin arrange for an appointment to see a medical practitioner to be made for the patient. This typically leads to an improvement in the medical care of the patient, as the onset of certain diseases or medical conditions may be predicted with the digital twin, such that the patient can be treated accordingly at an early stage, which not only is beneficial to the patient but can also reduce (treatment) costs. Moreover, major medical incidents that the patient may be about to suffer may be predicted by the digital twin based on the monitoring of the patient's sensor readings, thereby reducing the risk of such incidents actually occurring. Such prevention avoids the need for the provision of substantial aftercare following such a major medical incident, which also alleviates the pressure on a healthcare system otherwise providing such aftercare.

A digital twin may be used to simulate a new physical situation or state in a patient each time new information or data becomes available, e.g. when a parameter has changed as detected with a sensor or user input. The result is a new output variable field or distribution in a set of output parameters.

In some applications, it may be useful to update a digital twin in real time or intermittently based for example on sensor data such that it accurately represents a real physical state of the patient. This may be useful for example during surgery or other medical procedure during which real-time estimations of patient parameters would be of use.

However, such (almost) continuous runtime operation of a real-time digital twin - or multiple digital twins at the same time - requires considerable memory, storage, electrical energy, and computational power and time.

Non-digital-twin based approaches are known which can offer faster computation speed.

For example, methods are known that use the results of pre-generated static biomechanical organ models to account for intra-operative soft tissue deformation in image-guided surgery. The organ models simulate soft tissue deformation as a function of the anticipated surgical loading conditions e.g. gravity, device interactions such as an ultrasound probe or surgical tools, respiration, pharmacological response, oedema etc. During surgery, the organ surface deformation is measured and atlas-based or statistical shape model based image segmentation tools are applied to detect the shape correspondence between pre-computed and measured shapes. Once the best-fitting pre-computed shape has been detected, the pre-computed model results are used to predict the displacements of the internal points in the organ. With this result, a high contrast, high resolution pre-operative image is matched with (registered to) the intra-operative presentation.

These models are static and not updated in real time so as to accurately reflect the real-time state of the patients.

While these approaches address the problem of computation speed, there still remains a requirement for significant storage because each of the precomputed deformed shapes for the static model must be stored.

### SUMMARY OF THE INVENTION

Hence, there is a need for an improved approach to deriving physiological or anatomical parameters which can reduce memory and storage requirements, and which can still respond accurately and with high speed. The invention is defined by the independent claims. The dependent claims define advantageous embodiments.

According to examples in accordance with an aspect of the invention, there is provided a system, comprising
first processing means, arranged, in at least a first mode, to:
retrieve a digital model of at least part of an anatomy of a patient,
simulate a plurality of physical states of said at least part of the anatomy based on adjusting a set of one or more input parameters of the model and developing the digital model based on each set of adjusted parameters;
for each simulated physical state, derive one or more physiological or anatomical parameters associated with the simulated at least part of the anatomy, and store said derived parameters in a data storage arrangement along with the set of adjusted input parameters corresponding to the simulated physical state, to thereby build up a reference dataset; and
further processing means, the same as or different to the first processing means, arranged, in at least a second mode, to:
receive a set of one or more measured parameters pertaining to the at least part of the anatomy of the patient;
associate the measured patient parameters with one of the sets of model input parameters stored in the reference dataset and retrieve from the reference dataset the corresponding simulated physiological or anatomical parameters; and
generate an output based on said retrieved physiological or anatomical parameters.

Embodiments of the invention propose a two-stage process: a first stage in which a number of simulations are executed in advance on a personalized digital twin of at least part of an anatomy of a patient, and the results of the simulations stored; and a second stage in which these stored simulation results are subsequently used to provide real-time estimations of at least one patient parameter, for example during a medical procedure, e.g. surgery or catheter investigation.

The pre-stored simulation results can be output values of a critical physiological or anatomical parameter of the patient, and preferably one which cannot easily be directly measured for the patient during the procedure or action being carried out. These stored values alone consume less storage space than the full digital twin model, and require less processing resource to query than does execution of full simulations on the digital twin. Thus the pre-stored results effectively form a secondary 'lean' patient model which can be run for example locally in a tool or device which only has small memory and/or processing capacity, e.g. integrated locally at the distal end of a catheter.

This improved speed and reduced storage space comes at the cost of reduced flexibility in terms of the range of different parameters or measurements of the patient anatomy that can be derived. Hence, the lean model built up in the first stage of the proposed method is typically a specialized model, built up with the capability of providing fast output results for only a small set of, e.g. one or two or three, patient parameters. Hence different lean models may be generated for different specialized procedures, adapted for providing physiological or anatomical parameter results particularly important or pertinent to particular medical procedures or interventions.

The secondary lean model, i.e. reference dataset, referred to above can take a number of different forms. It may be simply a lookup table in some examples. In other examples it may be built as a response surface model. In either case, the lean model embodies a set of data representative of or corresponding to pre-generated simulation values of one or physiological or anatomical parameters for the patient, each based on a distinct set of input parameters for the digital twin model.

The digital model referred to above is preferably a personalized digital model, i.e. it is personalized to the anatomy or physiology of the patient in question. The digital model may be otherwise known as a digital twin. The digital model typically provides a model of both the elements and the dynamics of the at least portion of the anatomy of the patient (i.e. the physical twin). The digital twin may by way of example integrate artificial intelligence, machine learning and/or software analytics with spatial network graphs to create a 'living' digital simulation model of the at least portion of the patient's anatomy. By way of non-limiting example, the at least portion of the patient's anatomy may be a part of a lumen system of the patient (e.g. vascular or digestive systems), such that the digital twin comprises a model of this part of a lumen system of the patient. Such a living digital simulation may for example involve the use of a fluid dynamics model, a systemic model, a tissue deformation model and/or a fluid-structure interaction model in order to develop or update the digital twin based on received sensor data indicative of parameters of a physical state of the patient.

The first and second processing means may be the same as one another in some embodiments. For example, this means that the two processing means are provided or implemented by a single common processor or processor arrangement. Alternatively, the first and second processing means may be different to one another. For example, this means each is implemented or provided by a separate processing unit or processor arrangement.

The first processing means may be arranged to retrieve the digital model from a further data storage arrangement, different to the data storage arrangement on which the reference dataset is stored.

This means that the reference dataset can be stored on a small memory since it does not need to also store the digital twin model.

According to one or more embodiments, the second processing means may be different to the first and may be arranged communicatively coupled with the first data storage arrangement storing the reference dataset, and wherein said second mode operates independently of the first processing means.

This means that the first and second modes may be performed by separate units, e.g. building up the reference dataset can be done in advance by one unit, and the reference dataset can then be deployed by itself as a separate unit to provide a secondary 'lean' model. The lean model unit requires smaller storage space and less processing power.

In these embodiments, execution of the second mode is preferably also independent of the further data storage arrangement storing the digital model.

According to an alternative one or more embodiments, the first processing means may be arranged to retrieve the digital model from the same data storage arrangement on which the reference dataset is stored, and wherein the first mode further comprises, subsequent to building up the reference dataset, deleting the digital model from the data storage arrangement.

By deleting the digital model from the data storage arrangement before deployment of the second mode, the second mode can be run faster since the data stored on the data storage arrangement is less, for example. This provides an alternative to using separate data storage units for storing the digital twin model and storing the secondary lean model (the reference dataset).

In these examples, the first and further processing means may be the same. However, in other arrangements they may alternatively be different.

According to one or more embodiments, the first processing means may be further configured to generate said digital model based at least partly on image information of said at least part of the anatomy.

In these embodiments the system also performs the initial generation of the personalized digital model for the at least part of the patient's anatomy. For example, the first processing means may perform this step, or a different processing means may be provided to perform this step. Detailed explanation for implementing this step will be described later in this disclosure.

According to one or more embodiments, the further processing means may be arranged to receive the one or more measured parameters continuously or recurrently, and to run the second mode in real time with receipt of the measured parameters. The further processing means may actively obtain or retrieve the parameters continuously or recurrently in some examples (e.g. query or interrogate the source of the data, e.g. attached sensors or a server) or may be arranged to passively receive the data continuously or recurrently, and run the second mode in real time with their receipt.

The steps of the second mode may be run recurrently for example.

The measured patient parameters may be received in the form of sensor data from one or more sensors.

The system may include a communication module communicatively coupled to said further processing means and arranged to receive the sensor data. The data may pertain to one or more parameters relating to a physical state of said at least part of the anatomy of the patient. The system may in some embodiments include the one or more sensors for providing the patient parameter data.

By way of non-limiting example, the sensor data may include one or more of: medical image data of the at least part of the anatomy, blood pressure, heart rate, and tissue properties of the at least portion of the patient anatomy.

According to one or more embodiments, the output generated in the second mode may comprise a data output representative of the one or more retrieved physiological or anatomical parameters.

According to one or more embodiments, the second mode may further comprise determining, based on the retrieved one or more physiological or anatomical parameters, one or more medical actions pertaining to the patient. These may be recommended medical actions or interventions for taking responsive to the retrieved values of the parameter. They may relate to a change in an ongoing procedure for example. This feature will be described in more detail to follow.

As discussed above, the reference dataset may take different forms. The reference dataset may otherwise be referred to as a secondary model. In all examples it embodies a set of data representative of the one or more physiological or anatomical parameters derived in the first mode.

In some examples, the reference dataset may be in the form of a lookup table.

In some examples the reference dataset may take the form of a response surface model.

Examples in accordance with a further aspect of the invention provide a method comprising:
in a first phase:
   retrieving a digital model of at least part of an anatomy of a patient;
   simulating a plurality of physical states of said at least part of the anatomy based on adjusting a set of one or more input parameters of the model and developing the digital model based on each set of adjusted parameters;
   for each simulated physical state, deriving one or more physiological or anatomical parameters associated with the at least part of the anatomy, and storing said derived parameters in a data storage arrangement in association with the set of adjusted input parameters for the simulated physical state, to thereby build up a reference dataset; and
in a second phase:
   receiving a set of one or more measured parameters pertaining to the at least part of the anatomy of the patient;
   associating the measured patient parameters with one of the sets of model input parameters stored in the reference dataset and retrieving from the reference dataset the corresponding simulated physiological or anatomical parameters; and
   generating an output based on said retrieved physiological or anatomical parameters.

The method is preferably a computer-implemented method.

According to one or more embodiments, the method may further comprise generating said digital model based at least partly on image information of said at least part of the anatomy.

According to one or more embodiments, the one or more measured parameters may be received continuously or recurrently, and the steps of said second phase of the method are run in real time with receipt of the measured parameters.

Examples in accordance with a further aspect of the invention provide computer program product comprising code means configured, when run on a processor, to perform a method in accordance with any of the examples or embodiments outlined above or described below, or in accordance with any claim of this application.

Implementation options and details for each of the above steps may be understood and interpreted in accordance with the explanations and descriptions provided above for the apparatus aspect of the present invention (i.e. the system aspect).

Any of the examples, options or embodiment features or details described above in respect of the apparatus aspect of this invention (in respect of the system) may be applied or combined or incorporated into the present method aspect of the invention.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an example system in accordance with one or more embodiments;
Fig. 2 shows an example system in accordance with one or more embodiments;
Fig. 3 shows a further example system in accordance with one or more embodiments; and
Fig. 4 shows a block diagram of an example method in accordance with one or more embodiments.

### DETAILED DESCRIPTION OF THE EMBODIMENTS

The invention will be described with reference to the Figures. It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Embodiments of the invention provide a means for implementing a digital twin to provide estimated measurements of one or more physiological or anatomical parameters but in a way that requires less local storage space and local processing capacity. Embodiments are based on constructing and implementing a lean digital twin model or dataset by storing results of a number of full digital model simulations in advance and then using these pre-stored simulation results to provide physiological parameter estimations, in place of the full digital twin model.

From each pre-run model scenario, one or more key physiological or anatomical parameters can be extracted and stored, and the simulation then ended. This can be repeated for a range of varying input parameters for the model until a dataset is built up of values for the one or more parameters in a range of different possible scenarios. This limited dataset can then be deployed as a standalone lean model which can be queried in real time for example during a medical procedure, based on the measured parameters of the real physical situation. These can be tallied or registered against the records in the dataset to find a pre-stored scenario record which corresponds most closely to the current physical scenario. The corresponding results for one or more physiological parameter values can then be read off from the lean model data and provided as an output for example to a user or to a further device.

There is thus provided a method to apply a "lean" digital twin. The lean digital twin can make use for example of a lookup table or a response surface model or other data representation mode.

Advantages of this approach compared to known digital model methods include reduced power (energy) consumption, reduced memory and storage demands, and greater simplicity of operation. These advantages enable for example, and among other things, implementation in local devices and increased speed in availability of computational results. This enables for example near instantaneous response in terms of the output of the model.

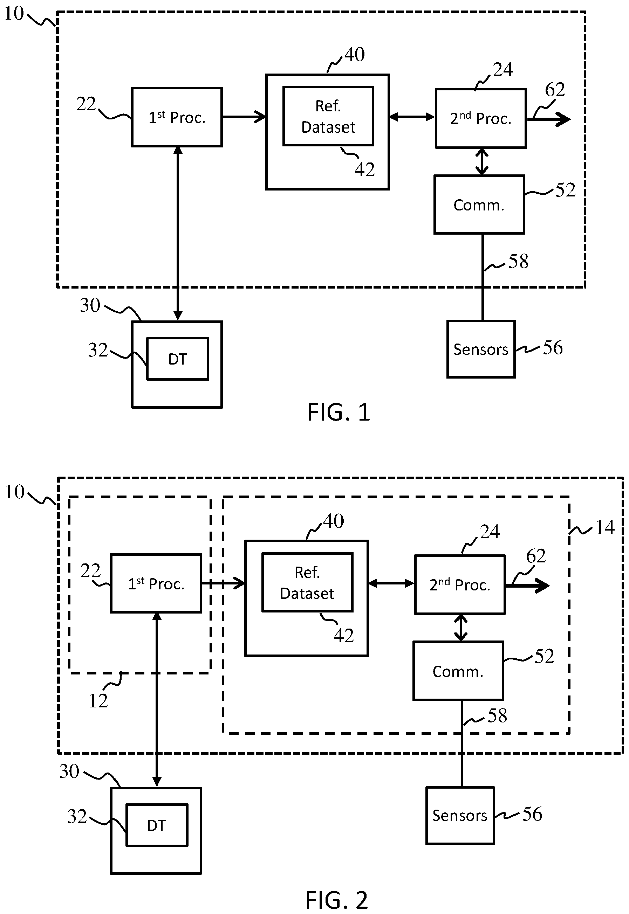

Fig. 1 schematically depicts one example system 10 in accordance with one or more embodiments of the invention. The components and operation of the system will first be briefly outlined, before describing implementation of the features in more detail.

The system 10 includes a first processing means 22. The first processing means is configured to execute a first mode or first operation of the system. The first mode or operation comprises retrieving a personalized digital model 32 ("DT") of at least part of an anatomy of a patient. The model is a digital twin of the at least part of the anatomy of the patient, and is configured to represent an accurate replica of the biophysical state of the at least part of the anatomy.

The digital model 32 may be retrieved from a data storage arrangement 30 arranged communicatively coupled in use with the first processing means 22. The data storage arrangement in Fig. 1 is shown as external to the system 10. However, in other examples, it may be included as part of the system.

The first mode further comprises simulating a plurality of physical states of said at least part of the anatomy of the patient based on adjusting a set of one or more input parameters of the digital model 32 and developing the digital model 32 based on each set of adjusted parameters. As will be described further below, the model has various parameters relating to the modelled anatomy which can be adjusted, and wherein adjusting these leads to altering of the simulated physical state of the anatomy which the model represents. The model input parameters may correspond to physical or physiological parameters of the modelled anatomy, and preferably may correspond to measurable patient parameters, e.g. vital signs, tissue structure and deformation, and parameters more specific to the anatomy part concerned, e.g. cardiac parameters in the case of the heart.

The range over which the input parameters are varied in order to simulate the range of scenarios may be user-determined or may be determined by the system, for example based on historical patient data indicative of the typical ranges over which those parameters have been observed, e.g. by sensors, to have varied in the past.

Once the model has been adjusted or developed in this way, for each simulated physical state, various output parameters of the modelled anatomy can be extracted, these representative for example of physiological or anatomical parameters of the at least portion of the anatomy of the patient being modelled.

In particular, from each of the plurality of simulated physical states, there is derived one or more physiological or anatomical parameters associated with the simulated at least part of the anatomy. These may be simply read off from the model in some cases, or may be extracted from the model, or the model may be interrogated or prompted to provide the parameters. These parameters may represent output parameters of the model. These may be parameters which cannot be, or cannot easily be, measured with direct methods such as sensors or medical imaging. By way of just one example, in the case of hemodynamic simulations for the cardiac anatomical region, the output parameter might include maximum wall shear stress.

The derived parameters are then stored in a data storage arrangement 40 along with the set of adjusted input parameters corresponding to the simulated physical state, to thereby build up a reference dataset 42. The reference dataset can take different forms. It may comprise simply a lookup table which links or associates the adjusted input parameters for each simulation with the derived output parameters for that state. The dataset may comprise a plurality of records, one record for each simulated physical state, and each record having two data fields, one data field representing the set of input parameters, and the other field representing the set of one or more derived physiological parameters. In further examples, the reference dataset may take the form of a response surface model, and will be further explained below.

In Fig. 1, the reference dataset is shown as stored in a data storage arrangement 40 which is part of the system 10. However, in other examples, the data storage arrangement 40 may be external of the system, and wherein the first processing means 22 is arranged in use to be communicatively coupled with the data storage arrangement 40. It is also noted that in some examples, the first 40 and second 30 data storage arrangements shown in Fig.1 may instead correspond to or be replaced by a single data storage unit, which may be external to the system 10 or may be part of the system.

The system further includes further (second) processing means 24. In Fig. 1, this is shown as a separate, second processing component 24. However, in further examples, the first 22 and second 24 processing means may be provided by a single processing component, e.g. a single central processor which performs the functions of both processing means. Hence the second processing means 24 may be the same as or different to the first processing means 22.

The second processing means is configured in use to perform a second mode or second system operation.

The second mode or operation includes receiving a set of one or more measured parameters pertaining to the at least part of the anatomy of the patient. These may be received for example from a set of one or more sensors 56 associated with the patient. By way of example, the second processing arrangement may be communicatively coupled with a communication module 52, wherein the communication module is communicatively coupled with the one or more sensors 56. The sensors may transfer sensor data 58 to the second processing means 24 (e.g. via the communication module), the sensor data being representative of the one or more measured parameters pertaining to the at least part of the anatomy. In further examples, the sensors may be coupled directly to the second processing means without the intermediate communication module.

By way of example, the sensor data may include one or more of: medical image data of the at least part of the anatomy, blood pressure, heart rate, and tissue properties of the at least portion of the patient anatomy.

In Fig. 1, the sensors are shows as external of the system 10. However, they may alternatively be part of the system 10 in further examples. The sensor data may be collected or received by the second processing means 24 continuously or recurrently, so that the measured patient parameters are constantly received at kept up to date. The second mode may be run in real time with receipt of the measured parameters in some examples. The steps of the second mode may be run recurrently with each receipt of new measured patient parameter data.

In addition to the sensor data, the measured patient parameters may also relate to broader medical parameters for the patient, such as current medication, or smoking behavior in some examples. These may be kept updated by a user (e.g. clinician) via a user interface for example, and received at the second processing means from such a user interface, or from a patient record database for example.

The measured patient parameters received at the second processing means are then associated with one of the sets of model input parameters stored in the reference dataset, and the corresponding simulated physiological or anatomical parameters are retrieved from the reference dataset. This means for example that each measured parameter is matched or registered to one of the model input parameters, e.g. the one that most closely matches in terms of the physical property it is representing. The set of those input parameters that then most closely match with the values of the received measured parameters is then identified in the reference dataset. The corresponding one or more physiological or anatomical parameters stored associated with that set of input parameters is then retrieved.

There is then generated an output 62 based on the retrieved physiological or anatomical parameters. This may in some examples simply comprise a data output representative of the one or more retrieved physiological or anatomical parameters.

Various features of the components and operation of the system will now be described in more detail.

As discussed above, embodiments of the invention make use of a personalized digital biophysical model of at least part of the anatomy of the patient. This is used to construct the reference dataset (or secondary lean model) based on running multiple simulations on this full digital model for a range of different model input parameters and storing the results. The digital model in the remainder of this application may also be referred to as a digital twin of the patient. The general principles of the digital twin and its implementation will now be briefly discussed.

The digital twin typically provides a model of both the elements and the dynamics of the at least portion of the anatomy of the patient (i.e. the physical twin). The digital twin may by way of example integrate artificial intelligence, machine learning and/or software analytics with spatial network graphs to create a 'living' digital simulation model of the at least portion of the patient's anatomy. By way of non-limiting example, the at least portion of the patient's anatomy may be a part of a lumen system of the patient (e.g. vascular or digestive systems), such that the digital twin comprises a model of this part of a lumen system of the patient. Such a living digital simulation may for example involve the use of a fluid dynamics model, a systemic model, a tissue deformation model and/or a fluid-structure interaction model in order to develop or update the digital twin based on adjustments of one or more input parameters of the model, representative of parameters of the physical state of (at least portion of the anatomy of) the patient.

The biophysical model 32, i.e. the digital twin, of the patient may be initially developed from patient data, e.g. imaging data such as CT images, MRI images, ultrasound images, and so on. This may be done in advance of executing the first mode or phase of the method in which the reference dataset is constructed.

For example, a medical scan may be conducted of the patient, and/or a set of one or more physiological or anatomical parameter measurements taken for the patient, and the digital model constructed based on this.

By way of example, a typical workflow for creating and validating a 3D, subject-specific biophysical model is depicted in "Current progress in patient-specific modeling", by Neal and Kerckhoff, 1, 2009, Vol. 2, pp. 111-126. For example, in case of a digital twin representing part of the cardiovascular system of the patient, such a biophysical model may be derived from one or more angiograms of the patient.

Development and implementation of digital twin models for various example applications are described in the literature for this field. By way of example, implementation details for various example digital twin models are described in the following papers: Gonzalez, D., Cueto, E. & Chinesta, F. Ann Biomed Eng (2016) 44: 35; Ritesh R. Rama & Sebastian Skatulla, Towards real-time cardiac mechanics modelling with patient-specific heart anatomies, Computer Methods in Applied Mechanics and Engineering (2018) 328; 47-74; Hoekstra, A, et al, Virtual physiological human 2016: translating the virtual physiological human to the clinic, interface Focus 8: 20170067; and "Current progress in patient-specific modeling", by Neal and Kerckhoff, 1, 2009, Vol. 2, pp. 111-126. Details are also outlined in "Computational Biomechanics for Medicine", Grand R. Joldes et al, Springer.

The fundamentals of a patient-specific digital model for a given patient's anatomy may be developed in advance of execution of the first mode (build-up of the reference dataset), such that before the procedure begins, the digital model is an accurate representation of the current physical state of the portion of the anatomy of the patient of interest, and incorporates sufficient information and knowledge about the material properties and physical response characteristics to allow the model to be dynamically evolved or developed by adjusting model input parameters.

In general, the digital model, e.g. of an organ or tissue area of the patient, incorporates a number of different (e.g. heterogeneous) material properties as parameters of the model, which may include blood vessels, muscles, fat, lining tissue, bones, calcified areas, which each have specific (biomechanical) material properties. These material properties form parameters for the model which can be adjusted to thereby alter the physical state which is being simulated by the model. Often, these model parameters reflect measurable physiological or anatomical parameters of the patient or their anatomy. In many example applications, these parameters are kept updated with real-time measured values for the parameters of the patient so that the model is continuously kept as a 'live' accurate replica of the state of the patient's anatomy.

However, in embodiments of the present invention, instead the model is constructed based on an initial state of the patient and then the input parameters are artificially adjusted through a range of different values to simulate a range of possible artificial physical states of the at least part of the patient anatomy modelled by the digital model.

For each simulated physical state, one or more physiological or anatomical parameters of the modelled anatomy can be extracted or read off from the model. These may advantageously be parameters which are not directly measurable using sensors in real time, so that the model provides an insight into physical parameters beyond those that can be measured using standard sensors or imaging equipment.

By way of non-limiting example, example input parameters of the model which may be adjusted may include one or more of: blood pressure; heart rate; breathing rate; diet; movement of skin or an outer surface of the body e.g. due to breathing or body movement (e.g. measured by medical imaging or movement sensors); subcutaneous tissue movement or deformation, e.g. due to interaction with medical instruments (e.g. measured by medical imaging); and medical instrument position or angle relative to the skin. Model input parameters may also include parameters which are not directly measurable, and/or which need to be estimated based on other measurements or data such as for instance blood viscosity. The model input parameter representative of blood viscosity may for example be a reference value which varies for instance depending upon medication.

By way of non-limiting example, example output parameters of the digital model which may be derived or extracted or read off to provide the one or more physiological or anatomical parameters may include one or more of: tissue wall shear stress; radiation dose distributions; internal organ tissue deformation; instrument position relative to a target e.g. a tumor; instrument position relative to a critical structure e.g. a nerve or a blood vessel. Instruments in this context may refer to for example surgical or interventional tools (hardware) such as knives or needles, or fields delivered by instruments such as thermal (radiofrequency or microwave ablation), electrical, acoustic (high intensity focused ultrasound), or ionizing radiation (radiotherapy).

The one or more physiological or anatomical parameters extracted from the model for each simulated state are then recorded in the reference dataset in association with the model input parameters to which they correspond. In the second mode, estimations of the one or more output parameters can be determined based on actual measurements of one or more patient parameters, these being mapped onto one or more of the input parameters stored in the reference dataset, and retrieving the corresponding recorded physiological or anatomical parameter(s).

As mentioned above, the reference dataset can take different forms.

In some examples, the reference dataset may comprise a lookup table.

In other examples, the reference dataset may take the form of a response surface, or response surface model (RSM). A response surface is a way of representing the relationships between one or more explanatory (input) variables and one or more response (output) variables. In this case, the input variables are the one or more model input parameters which may be varied between different of the simulations. The output variables are for example the one or more physiological or anatomical parameters which are extracted from each simulated model state, or model output parameters indicative of these physiological or anatomical parameters.

By way of clarification different terminology exists in the field for the class of models referred to in this disclosure as response surface models. These include for example approximation models, surrogate models, response surface models, and metamodels. The expression response surface model is intended to encompass any of these class of model.

Construction of a suitable response surface model for permitting lookup of stored physiological or anatomical parameter values based on measured or estimated input parameters will be apparent to the skilled person in this field. By way of example, the book Meyers RH, Montgomery DC, Anderson-Cook CM, Response Surface Methodology: Process and Product Optimization Using Designed Experiments, 4th edition, 2016, details methods for constructing and implementing surface response models suitable for application in embodiments of the present invention.

Reduced order modelling (ROM) is another suitable approach for providing the reference dataset in further examples.

As detailed above, once the physiological or anatomical parameter(s) corresponding to the measured patient parameters are retrieved from the reference dataset 42, an output 62 is generated based on this. In some cases, this output may be a data output representative of the retrieved parameter(s).

According to some examples, a step may be performed of interpreting or analyzing the retrieved one or more physiological or anatomical parameters. For example, there may be continuously or recurrently calculated a risk parameter based on historical data and on the newly derived values of the physiological or anatomical parameter. The risk parameter may in examples be compared with a defined threshold or other criterion, and an output generated based on this comparison. In some examples, there may be predicted an evolution of a risk parameter based on stored information pertaining to historical behavior of the patient. These interpretation or analysis steps may be performed by the second processing means 24 or may be performed by a different processing means in other examples.

The derived physiological parameter and/or said risk parameter may be recurrently or continuously output to a user interface in some examples. The user interface may include a display on which the output parameter(s) may be displayed, such that a user, e.g. the patient, nurse or physician, may use the information to inform decision making. For example, the user may decide to arrange one or more of an appointment, test or intervention.

As briefly discussed above, there are different options for the physical configuration of the first 22 and second 24 processing means and the first 30 and second 40 data storage arrangements. According to one or more advantageous embodiments, the first and second processing means may be provided as separate components.

Fig. 2 shows one advantageous example arrangement having this configuration. In this example, the first processing means is provided within a first unit 12 and the second processing means within a second unit 14. The reference dataset 42 is also stored in a data storage arrangement comprised by the second unit 14. The digital twin 32 stored on the other data storage arrangement may be part of the first unit 12 or may be outside of the first unit. During the first mode, the first and second processing means may be communicatively coupled.

Optionally, after the first mode, the two processing means may be disconnected, and the second mode may be operated independently of either the first processing means 22 or the other data storage arrangement 30 storing the digital model 32. By way of example, the second unit may be comprised as a local component of for example a medical tool, e.g. a catheter incorporating one or more sensors. By providing the second processing means in a separate unit to the first, the size of the processor required in the second unit 14 can be reduced. By also including only the first data storage arrangement 40 comprising the reference dataset and not the second data storage arrangement storing the whole digital model, the amount of storage space required on the second unit can be greatly reduced. This means the dimensions or form factor of the second unit can be reduced, allowing it to be incorporated for example as a local component in a medical device or tool or other peripheral unit.

As mentioned above, according to one or more examples, the first 22 and second 24 processing means may be implemented or provided by a single processor component which performs the functions of both. Additionally or alternatively, the first and second data storage arrangements may be provided by a single storage component in some examples.

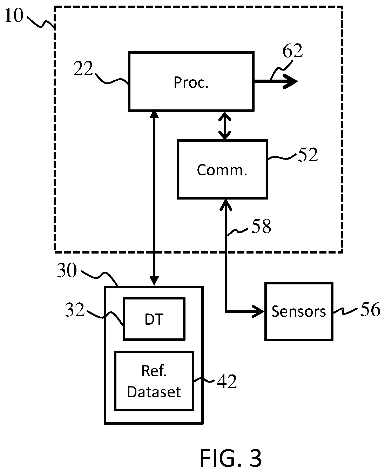

An example system 10 according to one or more embodiments is shown in Fig. 3 which includes both of these options. Here a single processing component 22 is provided, and single data storage arrangement 30. The data storage arrangement may be external to the system, as shown in Fig. 3. Alternatively, it may be comprised as part of the system in other examples. Where it is part of the system, preferably, the first mode or operation discussed above may further include a step of deleting or otherwise removing the stored digital model 32 from the data storage arrangement after completing the building up of the reference dataset 42. This then greatly reduces the amount of data stored on the data storage arrangement, which may allow the second mode (involving retrieval of data from the reference dataset) to operate with greater speed.

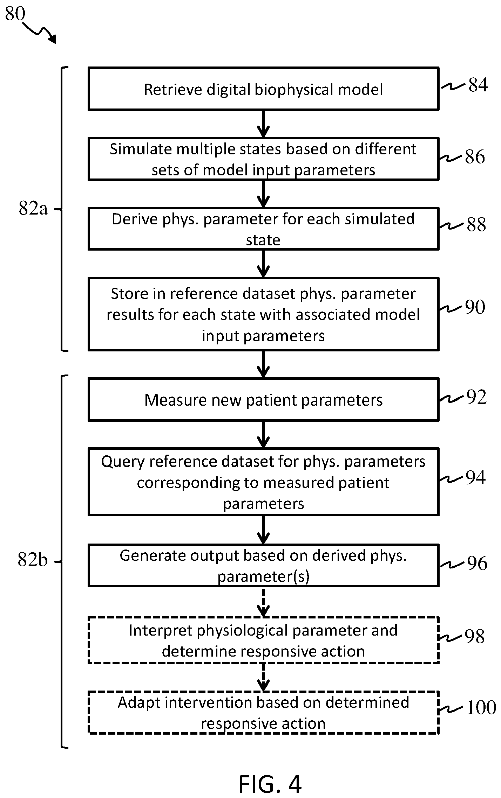

An example method 80 in accordance with one or more embodiments of the invention is outlined in block diagram form in Fig. 4. The method is preferably a computer implemented method, for implementation by one or more processors or computers.

The method 80 comprises a first phase 82a and a second phase 82b. The first and second phase of this method correspond to the first and second mode of the system discussed above.

The first phase 82a of the method 80 includes retrieving 84 a personalized digital model for a patient. The model may be retrieved from a local or external data store or memory for example.

The method further comprises simulating 86 a plurality of physical states of said at least part of the anatomy (i.e. a variety of scenarios for the model) based on adjusting a set of one or more input parameters of the model and developing the digital model based on each set of adjusted parameters.

The method further comprises, for each simulated physical state, deriving 88 one or more physiological or anatomical parameters associated with the at least part of the anatomy from the simulated model state. This may comprise deriving the one or more physiological or anatomical parameters from one or more direct output parameters of the model. In other cases, the directly measurable output parameter(s) of the model may constitute the one or more physiological or anatomical parameters to be derived from the model.

The method then further comprises storing 90 said derived parameters in a data storage arrangement along with the set of adjusted input parameters for the simulated physical state, to thereby build up a reference dataset. By way of non-limiting example, the reference dataset may take the form of a look-up table or a response surface model for instance. It may take the form of a database or any other mode or type of data storage representation, representing the data in any other way.

The method further includes a second phase 82b, performed after completion of the first phase 82a. In some examples, the first phase may be performed once to build up the reference dataset, and then the second phase may be performed recurrently as new data is collected pertaining to the patient, based on the use of the same reference dataset compiled in the first phase. In other examples, the first phase may also be performed recurrently, so as to keep the data in the reference dataset up to date and reflective of a current state of the patient's anatomy.

The second phase 82b comprises receiving a set of one or more measured patient parameters. These may pertain to the at least part of the anatomy of the patient for example, or may pertain to the patient more generally for example.

The method comprises associating the measured patient parameters with one of the sets of model input parameters stored in the reference dataset. In other words, the measured patient parameter(s) are mapped onto the model input parameters. The reference dataset is queried to identify the stored set of one or more input parameters which most closely matches the measured values of the patient data, and then there is retrieved from the reference dataset the corresponding simulated physiological or anatomical parameters stored in the dataset for those input parameters.

The method then comprises generating an output 96 based on the derived one or more physiological or anatomical parameters. This may be for example a data output representative of the derived parameters, which output may be provided for instance to a display or other user interface or might be output to another processor or another module in the system for further processing and analysis.

According to one or more embodiments, the second phase of the method may comprise a further optional step 98 of interpreting or analyzing the derived one or more physiological or anatomical parameters, for instance to determine a medical significance or consequence of the value. This may comprise for instance determining a risk factor for the patient, as has been discussed above.

The second phase 82b of the method may also comprise a further optional step of determining a proposed corrective action to be taken in relation to the patient responsive to the interpretation or analysis of the derived parameter(s). This recommended corrective action may be output to a user interface device for display to a user in some examples.

In some cases, the second phase 82b of the method may comprise a further optional step 100 of causing adaptation of a medical action or intervention being taken in relation to the patient, for example a surgical or investigatory procedure. This might be the case for example in instances in which the system is coupled to a surgical robot which is autonomously performing a surgical or other procedure in relation to a patient. The action of the robot may be adapted responsive to the determined interpretation or analysis of the derived parameter(s).

According to one or more embodiments, the first phase 82a of the method may further include an initial step of generating or constructing the personal digital model (digital twin) of the at least portion of the anatomy of the patient, having a patient-specific geometry for the anatomy. This may be constructed for example based on received medical image data, e.g. an ultrasound or CT or MRI scan of the patient. Principles underlying construction of digital twins has been described in detail earlier in this disclosure.

Examples in accordance with a further aspect of the invention also provide a computer program product comprising code means configured, when run on a processor or computer, to perform a method in accordance with any of the examples or embodiments outlined above or below, or in accordance with any claim of this application.

A number of example applications of systems according to embodiments of the invention for particular use cases will now be described.

One example application of embodiments of the system and method of the invention is for monitoring (e.g. risk monitoring) of atherosclerosis. This will now be briefly explained.

A low wall shear stress (WSS) in the arteries is known to be linked with the formation of atherosclerosis (plaque in the arteries). The WSS distribution is not measurable, but it is accessible to computation using a computational fluid dynamics (CFD) simulation (e.g. with a finite element model) and a medical scan, e.g. CT. A digital twin (digital model 32) for a patient can thus be created embodying a CFD biophysical model for the patient capable of providing estimations of this WSS distribution.

Using this model, the first mode or phase can be executed as described above to construct the reference dataset. The reference dataset may take the form for example of a lookup table (LUT) or response surface model (RSM)

To construct the lookup table (LUT) or response surface model (RSM), the CFD-based digital model of the patient is used to calculate the WSS distribution as a function of the model input parameters such as heart rate, medication, diet and possibly other factors. For example, to generate the plurality of simulations, the input parameter of heart rate might be varied between 60 - 170 beats per minute (BPM) in steps of 10 BPM, and/or the medication may be varied between "on" or "off'. The different input parameters may be altered separately to generate the different simulations or may be altered in parallel to generate different simulations.

When the medication is "off', the resulting calculated blood viscosity has a high value. When the medication is "on", the resulting calculated blood viscosity has a low value. In each situation, a post-processing algorithm automatically selects a number of locations with low WSS (for example 5 critical locations), including the corresponding WSS values. In this case, a generated lookup table may consist of 24 rows x 5 columns (12 possible heart rate values x 2 possible medication values = 24 combinations of heart rate and medication; and 5 critical locations with corresponding WSS values). The full CFD-based digital twin is thus effectively reduced to only 24 x 5 = 120 data points, compared to for example a 1,000,000 degrees of freedom finite element model.

In use, according to this example, the system may in the second mode or phase continuously or recurrently receive measurements of the heart rate of the patient and collect information on medication and diet, for example via the user interface. With the LUT or RSM, the system may then continuously determine the WSS at the five critical locations as a function of the measured heart rate, medication etc. This can be done by querying the LUT or RSM with the received measurements to retrieve corresponding stored values for the WSS at the five critical locations.

By way of example, the second phase in this example may further include a step of determining a risk factor for linear plaque based on use for instance of a further linear plaque build-up model. For example, using the linear plaque build-up model, the system may continuously determine a risk parameter, R, based for instance on a time average wall shear stress, R = f WSS. The system may further compare R to a pre-defined risk threshold or risk criterion and report the result accordingly to the user via a user interface. By way of example, the closeness of the risk factor to the risk factor threshold may be indicated by traffic-light colorings, for example by showing a green, yellow or red icon in the graphical user interface.

A further example application of embodiments of the present invention is use in radiation therapy for the lung.

Minimizing inadvertent damage to healthy tissue is important in radiation therapy. The critical physiological parameter in this example is the overlap between the beam and the tumor to be irradiated, and the overlap between the beam and the healthy tissue or organs ("dose distributions"). The aim of radiotherapy is to deliver a high radiation dose to the tumor ("tumor control probability", TCP) while minimizing the radiation dose to healthy tissue and hence the risk of healthy tissue complications (normal tissue complication probability). Tumor motion due to breathing imposes a challenge. This is particularly so for example in lung cancer treatment, where movement of the relevant tissue is clearly unavoidable.

It is possible to predict tumor motion by calculation, for example with a finite element model of the thorax including the lung. Real time application of such a model during the radiation therapy is difficult, due to the size of the model that is required. Hence such an application can benefit from use of a "lean digital twin" (i.e. the constructed reference dataset) as provided by embodiments of the present invention,

A personal digital model of the thorax including lung for a patient may thus be generated which provides as an output parameter an overlap between the radiation beam and a tumor in the lung (a dose distribution, or, for example, more specifically TCP and NTCP values). The model may compute this output parameter based on observable movement of the skin at the outside of the thorax. The input parameters for the model might be for example breathing rate and breathing depth, from which the model algorithms are able to compute tissue movement and, from this tumor movement and so the overlap between the beam and the tumor and healthy tissue as a function of time.

The generated lookup table or response surface model (the reference dataset) may thus be built up based on running multiple simulations with different input values for the breathing rate and depth. The reference dataset then stores the different values for these input parameters along with the corresponding values for tumor and healthy tissue overlap calculated by the model.

In operation, the second mode of the system may then comprise, during the radiation therapy procedure, continuously or recurrently measuring movement of the skin surrounding the thorax, or continuously or recurrently measuring breathing rate and depth. The corresponding tumor overlap values (dose distributions) can then be continuously or recurrently retrieved from the reference dataset by querying it with these measured parameter values.

The lookup table or response surface model thus determines the overlap (or offset) between the beam(s) and the tumor. The patient or beam position may be adapted based on the determined overlap or offset parameter.

A further example application of embodiments of the present invention is use for needle biopsy procedures, for example in the lung.

Needle biopsy in the lung can be required for instance in cases of suspicious lung nodules. Reducing false negatives and avoiding nerve and vessel damage in such needle biopsy requires accurate prediction and monitoring of the needle pathway in the moving lung. One solution is continuous X-Ray imaging during the procedure. However, this exposes the patient, and also the physician conducting the procedure, to significant amounts of radiation. Furthermore, X-Ray images have only low resolution and so do not provide as much detail as would be desired (e.g. 3D imaging with high resolution would be ideal).

Simulation of the needle pathway in the deformable and moving tissue using for instance a digital twin model provides an attractive alternative to X-Ray imaging, since it avoids exposure of the patient to radiation, and the simulation is able to make use the high resolution pre-operative CT scan. The model can thus provide simulations of needle path at high resolution and precision.

However, simulation using a full digital model is typically too slow for real-time application during the biopsy procedure, due to the model size (in terms of data quantity). Hence, such an application can benefit from use of a "lean digital twin" (i.e. the constructed reference dataset) as provided by embodiments of the present invention.

A personal digital model of the thorax including lung for a patient may thus be generated which allows calculation of the distance between the projected trajectory of the needle tip and the nodule, in different situations (breathing patterns, needle insertion angle relative to the patient). The model may compute this output parameter based on observable movement of the skin at the outside of the thorax. The input parameters for the model might be for example breathing rate, breathing depth, needle insertion angle, from which the model algorithms are able to compute tissue movement and, from this the needle position as a function of time.

The lookup table or response surface model (the reference dataset) may thus be built up based on pre-run simulations, and links values of measurable breathing rate and depth with corresponding simulated values for the needle position.

Before the surgical procedure, the model may determine an optimal needle path and a recommended insertion location and angle. At the start of the surgical procedure, the patient and the needle are registered while the needle tip is positioned at the recommended insertion location. The movement of the skin may be measured continuously during the procedure (e.g. via breathing rate and depth). The lookup table or response surface model is then used to determine the distance between the projected needle tip trajectory and the tumor while the needle progresses towards the tumor. The needle angle may be adapted by the clinician based on this distance.

In accordance with any of the embodiments outlined in this disclosure, the reference dataset may take the form of a set of multiple lookup tables linked to one another and hierarchically organized. Depending on the required look-up speed and accuracy during the real-time operation, LUTs at different hierarchical levels can be used.

For example, LUTs at different hierarchical levels may include greater or fewer input parameters for each data entry, i.e. for each stored simulation result. For example, one LUT might be constructed by running simulations in which two model input parameters are varied through a range of values, and storing for each simulation one or more output parameters. A second LUT might then be constructed by running simulations in which three or four model input parameters are varied through a range of values. Further LUTs may include further different numbers of input or output parameters. The LUTs with a greater number of input parameters allow for more accurate and detailed interpretations or outputs, but at the cost of slower processing and lookup speed. The LUTs with fewer input parameters allow for fast and streamlined processing and lookup speed but at the cost of potentially less precise output results. Depending upon the required speed vs accuracy, an appropriate one of the hierarchy of lookup tables can be chosen.

A further potential advantage of including multiple LUTs is, in accordance with one or more examples, comparisons may be performed between outputs derived from two or more different of the connected LUTs (that are clustered together). This may provide for instance a means of validation of the output parameters (i.e. the differences between outputs should not be significant).

A further advantage of inclusion of more than one LUT is as a safety measure. Having multiple different lookup tables which can be called upon introduces a degree of redundancy in the system, providing backup LUTs for instance in the case of system attacks, or a system failure.

A further potential advantage of including multiple LUTs, for example hierarchically organized, is in the case that the one or more physiological or anatomical parameters stored as the output parameter(s) may potentially have different values for a given set of input parameter depending for instance on a particular threshold parameter which is applied or a subjective opinion. For instance, as explained above, the one or more physiological or anatomical parameters may be derived from model simulation output parameters based on processing the model output parameter or performing a calculation with the model output parameter. The result of this processing or calculation may depend upon the value of one or more thresholds that are set (threshold parameter). In this case, by way of example, multiple LUTs may be constructed for the same set of varying model input parameters and the same one or more resulting physiological or anatomical parameter(s), but each LUT corresponding to a different value for the thresholding parameter.

In use, depending upon the application a different one of the plural LUTs can be chosen, depending upon the threshold parameter that a user (e.g. clinician) chooses to apply.

In accordance with any of the embodiments outlined above, the first mode or phase may comprise generating multiple reference datasets (e.g. multiple LUTs or RSMs) specialized for use in different clinical applications, each generated using simulations run on the same patient specific biophysical model (digital twin).

In particular, different clinicians implementing different clinical procedures may be interested in different observations and parameters. Taking such specialization in account, specific reference datasets for the same digital twin can be calculated and generated.

Thus, in effect, this amounts to running the first phase or mode outlined above multiple times using the same patient digital model, to generate multiple different reference datasets. Each execution of the first mode or first phase may comprise running a different set of multiple simulations by adjusting a different set of model input parameters, those being most relevant to the particular clinical application in question. Each execution of the first phase or mode may comprise then extracting a different set of one or more physiological or anatomical parameters from each of the simulations, those being most relevant to the clinical application in question.

By way of example, the patient digital model may be a tissue mechanics model. Different specializations may make use of the tissue mechanics model in a different way. For example, an interventional radiologist may for instance generate a lookup table from the model for use in a lung needle biopsy procedure (as outlined above). A radiation oncologist may for instance generate a lookup table for use in lung radiation therapy, as also outlined above.

As discussed, embodiments of the invention involve, in a first mode or phase, running a series of simulations to simulate a plurality of physical states of said at least part of the anatomy, this based on adjusting a set of one or more input parameters of the model and developing the digital model based on each set of adjusted parameters. Output parameters of the model are then extracted for each simulation, and these stored along with the corresponding input parameters in a reference dataset.

In accordance with one or more sets of embodiments, this process may be adapted such that, instead of storing the parameter values for all of the run simulations, parameter values are only stored if they are determined to be pathological, i.e. if it is determined that they may lead to one or more medical conditions. This restricts the number of entries made in the reference dataset, which reduces data storage.

For example, where the physiological parameter extracted from the model for each simulation is wall shear stress (WSS), there may be set a pre-defined minimum WSS threshold, below which the parameter results will be discounted. Only the parameter values for simulations in which the derived WSS values are above the pre-defined threshold are then stored in the reference dataset.

A more complex approach may be considered, wherein very large numbers of simulations are run as part of the first phase or first mode, in which multiple different model input parameters are varied each across a broad range and/or a fine index of precision, and multiple different output parameters recorded from the model. Again, only those simulations for which the output parameters indicate pathology (i.e. a medical condition) are recorded in the reference dataset. The parameter values for the remainder of the scenarios are discounted. This means that the reference dataset effectively becomes a highly distilled or condensed representation of only the most clinically significant patient scenarios. In use, the reference dataset can be queried using measured patient parameters in real time. Where no match for the measured parameters are found, it may be concluded that there is no medical pathology. Where a match is found, the relevant model physiological parameters can be read off and used to inform clinical decision making.

As mentioned, multiple different model output parameters could be extracted for each simulation, for example wall shear stress, fractional flow reserve, blood pressure, and/or cardiac output.

In accordance with one or more embodiments, regular or continuous updating of the reference dataset may be performed in parallel with (in real time with) use of the reference dataset for deriving physiological or anatomical parameter values. For example, at regular intervals, the digital model (digital twin) may be updated with new patient data such that it reflects a current real-time state of the patient. The updated digital twin is then used to begin running a new series of simulations, and the look up tables updated based on the results of these new simulations. The DT may be updated and new simulations started for example every one minute. Running a full set of simulations to update the reference dataset may for example take 10 minutes in total. However, by updating the DT twin data more frequently (e.g. every minute), each new minute that passes, the reference dataset data is being updated with new data.

For example, timings of recurrent updating may run as follows.
- 0 minutes - update digital twin (DT) so that it reflects real-time state of patient.
- 0-10 minutes - start simulations on DT to generate an updated full set of data for updating the LUT tables from the DT.
- 1 minute - update the DT again based on new collected data so far.
- 1-11 minutes, start simulations to generate updated LUT tables.
- 11 minutes: Implement the updated LUT tables by querying them with real time measurements from patient (i.e. data collected at 11 minutes). So, if the clinician queries the reference dataset at t=11, then LUT tables generated using the data until t=0, are used. If reference dataset is queried at t=12m then LUT tables generated with more data (1 min additional data) compared to the previous case) are used.

In other words, in accordance with this set of examples, it is proposed to run parallel processes: one in which the LUTs are recurrently updated based on the latest data available, and another process in which the LUT tables are used to derive physiological or anatomical parameter results.

As discussed above, embodiments of the invention make use of one or more processing means to perform data processing. The one or more processing means may comprise or be constituted by one or more processors.

Such processors can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor arrangement may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A system (10), comprising
first processing means (22), arranged, in at least a first mode, to:
retrieve a digital model (32) of at least part of an anatomy of a patient,
simulate a plurality of physical states of said at least part of the anatomy based on adjusting a set of one or more input parameters of the model and developing the digital model based on each set of adjusted parameters;
for each simulated physical state, derive one or more physiological or anatomical parameters associated with the simulated at least part of the anatomy, and store said derived parameters in a data storage arrangement (40) along with the set of adjusted input parameters corresponding to the simulated physical state, to thereby build up a reference dataset (42); and
further processing means (22, 24), the same as or different to the first processing means, arranged, in at least a second mode, to:
receive a set of one or more measured parameters pertaining to the at least part of the anatomy of the patient;
associate the measured patient parameters with one of the sets of model input parameters stored in the reference dataset (42) and retrieve from the reference dataset the corresponding simulated physiological or anatomical parameters; and
generate an output (62) based on said one or more retrieved physiological or anatomical parameters.

2. The system (10) according to claim 1, wherein the first processing means (22) is arranged to retrieve the digital model (32) from a further data storage arrangement (30), different to the data storage arrangement (40) on which the reference dataset (42) is stored.

3. The system (10) as claimed in claim 2, wherein the further processing means (24) is different to the first processing means (22), and is arranged communicatively coupled with the first data storage arrangement (40) storing the reference dataset (42), and wherein said second mode operates independently of the first processing means (22).

4. The system (10) as claimed in any of claims 1-3, wherein the first processing means (22) is further arranged to generate said digital model (32) based at least partly on image information of said at least part of the anatomy.

5. The system (10) as claimed in any of claims 1-4, wherein the further processing means (24) is arranged to receive the one or more measured parameters continuously or recurrently, and to run the second mode in real time with receipt of the measured parameters.

6. The system (10) as claimed in any of claims 1-5, wherein the measured patient parameters are received in the form of sensor data (58) from one or more sensors (56).

7. The system (10) as claimed in claim 6, wherein the sensor data (58) includes one or more of: medical image data of the at least part of the anatomy, blood pressure, heart rate, and tissue properties of the at least portion of the patient anatomy.

8. The system (10) as claimed in any of claims 1-7, wherein said output (62) comprises a data output representative of the one or more retrieved physiological or anatomical parameters.

9. The system (10) as claimed in any of claims 1-8, wherein the further processing means (24) is further configured to determine, based on the retrieved one or more parameters, one or more medical actions pertaining to the patient.

10. The system (10) as claimed in any of claims 1-9, wherein the reference dataset (42) is in the form of a lookup table.

11. The system (10) as claimed in any of claims 1-9, wherein the reference dataset (42) takes the form of a response surface model.

12. A method (80), comprising:
in a first phase (82a):
retrieving (84) a digital model (32) of at least part of an anatomy of a patient;
simulating (86) a plurality of physical states of said at least part of the anatomy based on adjusting a set of one or more input parameters of the model and developing the digital model based on each set of adjusted parameters; and
for each simulated physical state, deriving one or more physiological or anatomical parameters associated with the at least part of the anatomy, and storing (90) said derived parameters in a data storage arrangement along with the set of adjusted input parameters for the simulated physical state, to thereby build up a reference dataset (42); and
in a second phase (82b):
receiving (92) a set of one or more measured parameters pertaining to the at least part of the anatomy of the patient;
associating the measured patient parameters with one of the sets of model input parameters stored in the reference dataset (42) and retrieving (94) from the reference dataset the corresponding simulated physiological or anatomical parameters; and
generating (96) an output based on said retrieved physiological or anatomical parameters.

13. The method (80) as claimed in claim 12, the method further comprising generating said digital model (32) based at least partly on image information of said at least part of the anatomy.

14. The method (80) as claimed in claim 12 or 13, wherein the one or more measured parameters are received continuously or recurrently, and the steps of said second phase (82b) of the method are run in real time with receipt of the measured parameters.

15. A computer program product comprising code means configured, when run on a processor, to perform the method (80) of any of claims 12-14.
